# EUROPÄISCHE PATENTSCHRIFT

(11) **EP 1 484 397 B1**
(45) Veröffentlichungstag und Bekanntmachung des Hinweises auf die Patenterteilung: **17.01.2007**
(21) Anmeldenummer: 04013790.3
(22) Anmeldetag: 28.03.2000
(51) Int. Cl.: C12N 15/11, C07K 14/47, A61K 48/00

(54) **Einem Peptid aus Antigen MUC-1 entsprechende DNS zur Auslösung einer Immunreaktion gegen Tumorzellen**
DNA corresponding to a peptide from antigen MUC-1 for triggering an immune response to tumor cells
ADN correspondant à un peptide dérivé de l'antigène MUC-1 destiné à déclencher une réaction immunitaire contre des cellules tumorales

(30) Priorität: 16.04.1999 DE 19917195
(43) Veröffentlichungstag der Anmeldung: 08.12.2004
(62) Teilanmeldung aus: 00926764.2
(73) Patentinhaber: Immatics Biotechnologies GmbH, 72076 Tübingen (DE)
(72) Erfinder: Brossart, Peter, Dr., 72762 Reutlingen (DE); Stevanovic, Stefan, Dr., 72074 Tübingen (DE); Brugger, Wolfram, Dr., 78050 Villingen-Schwenningen (DE); Kanz, Lothar, Prof. Dr., 72076 Tübingen (DE); Rammensee, Hans Georg, Prof. Dr., 72070 Tübingen (DE)
(74) Vertreter: Krauss, Jan

(56) Entgegenhaltungen:
- WO-A-00/06723
- WO-A-97/11715
- WO-A-98/50527
- DE-A- 19 516 673
- P BROSSART ET AL.: "Identification of HLA-A2-restricted T-cell epitopes derived from the MUC1 tumor antigen for broadly applicable vaccine therapies" BLOOD., Bd. 93, Nr. 12, 15. Juni 1999 (1999-06-15), Seiten 4309-4317, XP002147432 W.B.SAUNDERS COMPAGNY, ORLANDO, FL., US ISSN: 0006-4971

## Beschreibung

Die vorliegende Erfindung betrifft ein von dem MUC-1-Gen abgeleitetes Peptid, durch das eine HLA-A2-restringierte Immunreaktion gegen Tumorzellen auslösbar ist.

Brossart et al. zeigen in einem Abstract zu einem auf dem 40. jährlichen Treffen der American Society of Hematology, 1998, gehaltenen Vortrag die Möglichkeit auf, ein derartiges Peptid von dem MUC-1-Gen abzuleiten, ohne jedoch die Sequenz eines von ihnen getesteten Peptides oder die Lokalisation der Sequenz eines Peptides, das nicht von dem Tandem-Repeat-Bereich von MUC-1 abgeleitet ist, zu erwähnen.

Peptide zur Auslösung einer Immunreaktion gegen Tumorzellen sind bspw. auch in der Publikation von Finn, O.J., et al., (1995) "MUC-1 Epithelial Tumor Mucin-Based Immunity and Cancer Vaccines", Immunol Rev 145, Seiten 61 bis 89, erwähnt, allerdings ebenfalls ohne Angabe von Sequenzinformationen.

Unter einer Immunreaktion wird bspw. die Zerstörung von Zellen durch solche T-Zellen verstanden, die wegen ihrer Fähigkeit, andere Zellen abzutöten, auch als zytotoxische T-Zellen bezeichnet werden. Zur Auslösung einer derartigen Immunreaktion durch zytotoxische T-Zellen müssen den T-Zellen dazu von einem MHC-Molekül auf der Zelloberfläche fremde Proteine z.B. infolge einer Virusinfektion präsentiert werden.

Diese MHC-Moleküle sind Peptidrezeptoren, die normalerweise Peptide innerhalb der Zelle binden, um sie zu der Zelloberfläche zu transportieren, wo der Komplex aus Peptid und MHC-Molekül durch T-Zellen erkannt werden kann. In normalen Zellen werden die durch MHC-Moleküle gebundenen Peptide von den üblichen zelleigenen Proteine abgeleitet. Während ihrer Differenzierung in einem Organismus werden T-Zellen tolerant gegenüber Komplexen von eigenen Peptiden mit eigenen MHC-Molekülen. Somit kann jedes neue Peptid, das später erscheint, z.B. ein solches, das durch die Infektion mit einem Virus von einer Zelle produziert wird, von T-Zellen erkannt werden.

Es gibt zwei Klassen von MHC-Molekülen, von denen diejenigen, die mit zytotoxischen T-Zellen wechselwirken, der Klasse I angehören. Klassische humane Klasse I-Moleküle sind HLA-A, B und C, wobei HLA-A2 eine Unterklasse der HLA-A-Moleküle darstellt.

Ist das Zustandekommen einer Immunreaktion von dem Vorhandensein eines bestimmten MHC-Moleküles, z.B. HLA-A2, abhängig, so spricht man von einer MHC-restringierten, z.B. einer HLA-A2-restringierten Immunreaktion. Es gibt vereinzelt auch T-Zell-Reaktionen, die unabhängig von MHC-Molekülen stattfinden. Man spricht dann von MHC-unrestringierten Immunreaktionen.

In der eingangs erwähnten Publikation von Finn et al. wurde zur Behandlung eines Tumorpatienten vorgeschlagen, eine solche MHC-unrestringierte Immunreaktion gegen ein Glykoprotein auszulösen, das durch das Gen MUC-1 codiert wird und auf der Oberfläche von Zellen vorkommt. Ziel ist dabei das Hervorrufen einer effektiven Immunantwort mit lebenslanger Immunität.

Auch in der WO 97/11715 A1 und der WO 98/50527 werden von MUC-1 abgeleitete Peptide zur Auslösung einer Immunreaktion vorgeschlagen. Ferner werden in der WO 00/06723 A1 eine Reihe von MUC-1-abgeleiteten Peptiden offenbart, von denen jedoch nur einige wenige geeignet waren, eine Immunreaktion auszulösen.

Das durch das Gen MUC-1 codierte Protein, das ebenfalls MUC-1 genannt wird, wird von normalen Epithelzellen meistens polarisiert so exprimiert, dass es dem Immunsystem normalerweise nicht zugänglich ist, wie bspw. im Darm, wo es auf der apikalen Seite der Epithelzellen, d.h. ins Darmlumen hineinragend exprimiert wird.

Auf Tumorzellen wird dagegen MUC-1 nicht polarisiert exprimiert, sondern bedeckt die gesamte Zelle. Das Level der Expression ist bei Tumorzellen höher als bei normalen Zellen, aber die Moleküle liegen bei Tumorzellen unvollständig glykosiliert vor, d.h. die Seitenketten aus Zuckermolekülen weisen eine verkürzte Struktur gegenüber MUC-1 auf, das von gesunden Zellen hergestellt wird. Die unvollständige Glykosilierung führt dazu, dass Epitope auf dem Protein-Anteil von MUC-1, die normalerweise durch Zucker-Seitenketten maskiert vorliegen, auf Tumorzellen dem Immunsystem zugänglich sind. Somit kann das Immunsystem dieses unterglykosilierte MUC-1 von normalem, nicht unterglykosiliertem MUC-1 unterscheiden.

Neben den Seitenketten aus Zuckermolekülen besteht ein weiteres wesentliches Strukturmerkmal von MUC-1 in den sogenannten Tandem-Repeats. Dabei handelt es sich um Abfolgen aus jeweils 20 Aminosäureresten, die sich in identischer oder ähnlicher Weise ca. 20 bis 125 Mal in dem MUC-1-Molekül wiederholen.

Zur Auslösung einer effektiven Immunreaktion durch T-Zellen sind nun aber Co-Stimulatoren erforderlich, zu denen insbesondere die sogenannten dendritischen Zellen zählen, die von außen angebotene Proteine aufnehmen und nach deren Prozessierung zu Peptiden so präsentieren können, dass zytotoxische T-Zellen dadurch aktiviert werden können. Finn et al. schlagen daher vor, synthetische Peptide, die die MUC-1-Tandem-Repeats repräsentieren, zusammen mit einem Adjuvans, das dendritische Zellen anlockt, zu injizieren. Bei einem Adjuvans handelt es sich um eine Substanz, die bei gemeinsamer Injektion mit einem Stoff, gegen den eine Immunreaktion ausgelöst werden soll, die Antwort des Immunsystems auf diesem Stoff unspezifisch verstärkt. Durch eine derartige Impfung soll eine MHC-unrestringierte T-Zellreaktion gegen die Tandem-Repeats erhalten werden. Es wird sogar vorgeschlagen, in den synthetischen Peptiden Aminosäuren, die eine MHC-Klasse-I-Bindung vermitteln könnten, durch andere zu ersetzen, damit keine MHC-restringierten T-Zellen durch eine Präsentation der Peptide durch MHC-Klasse-I-Moleküle entstehen können.

Einerseits kann durch die Verhinderung einer MHC-restringierten Immunantwort das Problem einer möglichen Autoimmunität verringert werden, andererseits besteht ein großer Nachteil in der geringeren Effizienz einer MHC-unrestringierten Immunantwort gegenüber einer MHC-restringierten. Somit sind auch die Chancen, mit einer MHC-unrestringierten Immunantwort einen Tumor zum Verschwinden zu bringen, geringer als mit einer MHC-restringierten Immunantwort.

Die Erfinder der vorliegenden Anmeldung haben daher anlässlich des eingangs erwähnten Vortrages vorgeschlagen, eine MHCrestringierte Immunantwort gegen MUC-1-exprimierende Zellen auszulösen. Sie berichten, dass hierzu die bekannte MUC-1-Aminosäuresequenz mittels einer Computeranalyse nach HLA-A2-bindenden Motiven abgesucht wurde. Peptide mit hoher Bindungswahrscheinlichkeit wurden identifiziert, synthetisiert und daraufhin untersucht, ob sie mit Hilfe von dendritischen Zellen in vitro zytotoxische T-Zellen induzieren können. Die so erzeugten zytotoxischen T-Zellen entwickelten eine Antigen-spezifische HLA-A2-restringierte zytotoxische Aktivität gegen diejenigen Zielzellen, die zuvor mit dem jeweiligen Peptid inkubiert wurden, mit dem die zytotoxischen T-Zellen aktiviert wurden, oder gegen MUC-1-exprimierende Tumorzellen.

Vor diesem Hintergrund ist es Aufgabe der vorliegenden Erfindung, ein weiteres Peptid und/oder für das Peptid kodierende Nukleinsäuren für eine Verwendung bereitzustellen, durch welche eine Immunreaktion gegen Tumorzellen ausgelöst werden kann.

Diese Aufgabe wird erfindungsgemäß gelöst durch die Bereitstellung des Peptids mit der SEQ ID-Nr.: 2 aus dem beigefügten Sequenzprotokoll und/oder einer der für das Peptid kodierenden Nukleinsäure zur Auslösung einer Immunreaktion.

Die der Erfindung zugrundeliegende Aufgabe wird auf diese Weise vollkommen gelöst.

Die nicht vorveröffentlichte WO 00/06723 erwähnt ein Peptid mit der Sequenz SEQ-ID-Nr. 2, identifiziert dieses Peptid jedoch als nicht für eine Verwendung zur Auslösung einer Immunreaktion im Zusammenhang mit einer Tumortherapie geeignet.

Mit Hilfe des erfindungsgemäßen Peptides können zytotoxische T-Zellen generiert werden, die eine Antigen-spezifische MHCrestringierte zytotoxische Aktivität gegen MUC-1 exprimierende Tumorzellen entwickeln und diese zerstören.

In einer weiteren bevorzugten Ausgestaltung betrifft die Erfindung

die *ex vivo* Verwendung einer erfindungsgemäßen Nukleinsäure im Rahmen einer Gentherapie.

Dabei kann die Gentherapie in Form der oben bereits beschriebenen Transformation von Antigen-präsentierenden Zellen, insbesondere dendritischen Zellen, erfolgen, die oder deren Vorläuferzellen zuvor aus dem Körper eines zu behandelnden Organismus entnommen wurden, um nach der Transformation wieder in den Körper eingebracht zu werden. Gegenüber der Verwendung von Peptiden ist dabei, wie bereits erläutert, die längeranhaltende Präsentation von Vorteil.

Eine weitere Möglichkeit besteht darin, die Nukleinsäure so in den Körper einzubringen, dass sie selektiv von Antigen-präsentierenden Zellen, insbesondere dendritischen Zellen, aufgenommen und exprimiert wird. Diese Verwendung hat den Vorteil, dass außer der Verabreichung keine weiteren Maßnahmen, wie bspw. die Anzucht und selektive Vermehrung entnommener dendritischer Zellen bzw. deren Vorläuferzellen, notwendig sind.

Die Erfindung betrifft ferner die Verwendung einer erfindungsgemäßen Nukleinsäure zur Transformation oder Transfektion von Zellen *in vitro*. Der Einsatz der Nukleinsäure *in vitro* hat den Vorteil, dass Verfahren, wie bspw. die Elektroporation und/oder Hilfsstoffe wie bspw. Calciumphosphat oder DEAE-Dextran, verwendet werden können, die die Aufnahme von Nukleinsäuren in Zellen wesentlich erleichtern und verbessern, die aber in vivo nicht einsetzbar sind.

Dabei können, wie bereits erwähnt, Antigen-präsentierende Zellen, insbesondere dendritische Zellen, behandelt werden, die bzw. deren Vorläuferzellen aus einem Patienten entnommen wurden und die später wieder in den Patienten eingebracht werden.

Ein weiterer Aspekt der vorliegenden Erfindung ist die Verwendung mindestens eines erfindungsgemäßen Peptides oder einer erfindungsgemäßen Nukleinsäure zur Auslösung einer Immunreaktion im Zusammenhang mit einer Tumortherapie oder mit einer gegenüber der Entstehung eines Tumors vorbeugenden Behandlung. Vorteilhaft ist dabei, dass die bei einer Tumorerkrankung häufig beobachtete Immunsuppression und Toleranz gegenüber MUC-1 durch die erfindungsgemäße Verwendung der Peptide oder Nukleinsäuren umgekehrt werden kann. Darüber hinaus kann die erfindungsgemäße Verwendung zusätzlich zu etablierten Tumortherapien eingesetzt werden.

Eine vorbeugende Behandlung ist vor allem bei Personen vorteilhaft, die, bspw. erblich bedingt oder weil sie früher bereits einen Tumor hatten, ein erhöhtes Risiko haben, an einem Tumor zu erkranken.

In einer Weiterbildung erfolgt die Verwendung der erfindungsgemäßen Peptide oder Nukleinsäuren in Kombination mit einem Pan-HLA-DR-bindenden Peptid.

Vorteilhaft bei der Verwendung eines solchen Pan-HLA-DR-bindenden Peptides ist, dass die Antigen-spezifische zytotoxische Aktivität der generierten T-Zellen verstärkt wird.

In einer Weiterbildung wird mindestens eines der erfindungsgemäßen Peptide oder Nukleinsäuren zusammen mit Antigen-präsentierenden Zellen, insbesondere dendritischen Zellen, inkubiert und erst dann in einen Organismus eingebracht, aus dem die Antigen-präsentierenden Zellen oder deren Vorläuferzellen zuvor entnommen wurden.

Der Vorteil dieses Verfahrens besteht darin, dass der Erfolg beim Auslösen einer Immunreaktion auf diese Art und Weise gesicherter und kontrollierbarer ist als bei der Injektion eines peptides zusammen mit einem Adjuvans, bei der die Immunreaktion einmal stärker und einmal schwächer ausfallen kann. Gerade bei einer Tumortherapie sollte man jedoch den Erfolg beim Auslösen einer Immunreaktion sicherstellen können, um nicht durch eine uneffektive Behandlung möglicherweise wertvolle Zeit zu verlieren.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung werden in der nachfolgenden Beschreibung dargestellt und erläutert.

### Beispiel 1 Induktion von Antigen-spezifischen zytotoxischen T-Zellen unter Verwendung von Peptiden und dendritischen Zellen

### 1.1 Bereitstellung von dendritischen Zellen

Wie bspw. bei Brossart, P., et al., Cancer Res 58 (1998), Seiten 732 ff., beschrieben, wurden zunächst mononukleäre Zellen aus peripherem Blut durch Ficoll/Paque (Gibco-BRL, Grand Island, NY) Dichtegradienten-Zentrifugation aus buffy coat-Präparationen von heparinisiertem Blut dreier gesunder Spender isoliert. Die Zellen wurden in Zellkulturschalen in RP10-Medium ausgesät. Nach zwei Stunden bei 37°C wurden die nicht anhaftenden Zellen entfernt und die anhaftenden Blutmonozyten in RP10-Medium kultiviert, das folgende Zytokinzusätze enthielt: humanes rekombinantes GM-CSF (Leukomax, Sandoz, 100 ng/ml), IL-4 (Genzyme, 1000 IU/ml), und TNF-α (Genzyme, 10 ng/ml). Nach siebentägiger Kultivierung zeigten die Zellen in der FACS-Analyse eine starke Expression der für den Phänotyp reifer dendritischer Zellen charakteristischen Oberflächenmarker MHC Klasse I und II, CD83, CD80, CD86, CD40 und CD54 und wurden somit als dendritische Zellen identifiziert.

### 1.2 Identifizierung geeigneter Peptide

Zur Identifizierung von Peptiden, die mit einer hohen Wahrscheinlichkeit durch HLA-A2 präsentiert werden, wurde zunächst die durch das Gen MUC-1 codierte Aminosäuresequenz mit Hilfe eines Computerprogrammes auf potentiell HLA-A*0201 (ein bestimmter HLA-A2-Typ) bindende Peptid-Motive abgesucht, wie es bspw. in Rammensee, H.G., et al., Landes Bioscience, Austin, Texas, USA (1997), beschrieben ist.

Die ausgesuchten Sequenzen und Kontrollsequenzen sowie ein PAN-HLA-DR-bindendes Peptid wurden unter Verwendung von Standard Fmoc-Chemie auf einem Peptidsynthesizer synthetisiert.

### 1.3 Induktion zytotoxischer T-Zellen

5 x 10⁵ dendritische Zellen aus 1.1. wurden zwei Stunden lang mit 50 µg/ml synthetischem Peptid aus 1.2 inkubiert, gewaschen und mit 2,5 x 10⁶ autologen, d.h. von denselben Spendern abstammenden, mononukleären Zellen aus peripherem Blut in RP10-Medium inkubiert. Die dendritischen Zellen wurden teilweise zusätzlich zu den Kontrollpeptiden und den potentiell HLA-A2-bindenden Peptiden mit 50 µg/ml eines PAN-HLA-DR-bindenden Peptides inkubiert, das ein T-Helfer-Epitop darstellt.

Nach siebentägiger Kultivierung wurden die Zellen erneut stimuliert, indem autologe, mit dem synthetischen Peptid aus 1.2 vorinkubierte mononukleäre Zellen aus peripherem Blut zugefügt wurden. Am ersten, dritten und fünften Tag wurde jeweils 1 ng/ml humanes rekombinantes IL-2 (Genzyme) zugesetzt. Das Zytokin IL-2 ist zur Kultivierung und Vermehrung von zytotoxischen T-Zellen notwendig. Insgesamt wurde bis zu dreimal in wöchentlichem Abstand stimuliert.

### 1.4 Nachweis zytotoxischer T-Zell-Aktivität

T2-Zellen, die sich dadurch auszeichnen, dass sie HLA-A2 herstellen, aber über diese MHC-Klasse-I-Moleküle keine selbst synthetisierten Peptide präsentieren können, wurden zwei Stunden lang mit 50 µg/ml von synthetischem Peptid aus 1.2 vorinkubiert und eine Stunde lang bei 37°C mit [⁵¹Cr]-Natriumchromat in RP10 markiert. Jeweils 10⁴ dieser Zellen wurden in je eine Vertiefung einer 96-Well-Platte übertragen. Zuvor nach 1.3 generierte zytotoxische T-Zellen wurden zugesetzt, so dass jeweils ein Endvolumen von 200 µl pro well erreicht wurde. Anschließend wurde vier Stunden bei 37°C inkubiert. Wenn nun die zytotoxischen T-Zellen die zuvor mit ⁵¹Cr markierten Zellen abtöten, so lysieren sie diese Zellen, und das in den Zellen enthaltene ⁵¹Cr wird freigesetzt. Die freigesetzte Radioaktivität wird am Ende des Versuchs in den abgenommenen Zellüberständen durch Zählen in einem β-Counter bestimmt. Somit erhält man ein Maß für die zytotoxische Aktivität der T-Zellen.

### 1.5 Peptide, die eine HLA-A2-restringierte Immunantwort auslösen

Zwei der synthetisierten, von der Sequenz des MUC-1-Genes abgeleitete Peptide waren in dem zuvor beschriebenen Verfahren in der Lage, zytotoxische T-Zellen mit Hilfe von dendritischen Zellen in vitro zu induzieren. Dabei handelt es sich um die beiden Peptide mit der Sequenz SEQ ID-Nr.: 1 und der Sequenz SEQ ID-Nr.: 2 aus dem Sequenzprotokoll. Die zytotoxischen T-Zellen, die unter Verwendung des Peptides mit der Sequenz SEQ ID-Nr.: 1 induziert wurden, konnten spezifisch T2-Zellen abtöten, die mit genau diesem Peptid vorbehandelt wurden, während sie nicht in der Lage waren, T2-Zellen abzutöten, die mit anderen Peptiden vorbehandelt waren. Umgekehrt waren zytotoxische T-Zellen, die unter Verwendung anderer Peptide induziert wurden, nicht in der Lage, T2-Zellen abzutöten, die mit dem Peptid mit der Sequenz SEQ ID-Nr.: 1 vorbehandelt waren. Entsprechend verhielt es sich mit zytotoxischen T-Zellen, die unter Verwendung des Peptides mit der Sequenz SEQ ID-Nr.: 2 induziert wurden. Diese Zellen konnten nur solche T2-Zellen abtöten, die mit dem Peptid mit der Sequenz SEQ ID-Nr.: 2 vorbehandelt waren.

Dabei zeigten zytotoxische T-Zellen, die in Gegenwart des PAN-HLA-DR-bindenden Peptides induziert wurden, eine höhere zytotoxische Aktivität als solche, die in Abwesenheit dieses Peptides induziert wurden.

### Beispiel 2 Lyse von Tumorzellen durch MUC-1-Peptid spezifische zytotoxische T-Zellen

Verschiedene Tumorzellinien, die wie durch FACS-Analyse nachgewiesen entweder sowohl HLA-A2 als auch MUC-1 oder jeweils nur eines der beiden Zelloberflächenmoleküle exprimieren, wurden mit ⁵¹Cr markiert, wie es unter 1.4 für T2-Zellen beschrieben wurde. Anschließend wurden die Zellen, wie ebenfalls unter 1.4 beschrieben, mit zytotoxischen T-Zellen inkubiert, die wie unter 1.3 beschrieben, induziert wurden.

Diejenigen T-Zellen, die unter Verwendung der Peptide mit der Sequenz SEQ ID-Nr.: 1 oder der Sequenz SEQ ID-Nr.: 2 aktiviert wurden, waren in der Lage, Tumorzellen der Linien MCF-7 (Brustkrebszellen), A-498 (Nierenzellkarzinomzellen) und MZ1774-RCC (Nierenzellkarzinomzellen), die HLA-A2 und MUC-1 exprimieren, zu lysieren. Sie konnten jedoch keine Zellen der Zellinien Croft (EBV-immortalisierte B-Zellen), die kein MUC-1 exprimieren, sowie Caki-2 (Nierenzellkarzinomzellen), SK-OV-3 (Eierstockkrebszellen) und K562 (proerythroblastische Zellen), die kein HLA-A2 exprimieren, lysieren.

Ebensowenig konnten die unter Verwendung der Peptide mit der Sequenz SEQ ID-Nr.: 1 und SEQ ID-Nr.: 2 aktivierten zytotoxischen T-Zellen die HLA-A2- und MUC-1-exprimierenden Nierenzellkarzinomzellen der Linie MZ1774-RCC lysieren, wenn die Nierenzellkarzinomzellen zuvor 30 Minuten lang mit 10 µg/ml eines gegen HLA-A2 gerichteten monoklonalen Antikörpers (BB7.2, Coulter-Immunotech) inkubiert wurden.

Diese Ergebnisse zeigen, dass die unter Verwendung der erfindungsgemäßen Peptide generierten zytotoxischen T-Zellen nur Tumorzellen abtöten, die sowohl HLA-A2 als auch MUC-1 exprimieren.

Die erfindungsgemäßen Peptide sind somit dazu geeignet, auch im Rahmen einer Therapie eine Immunreaktion gegen bestimmte Tumorzellen auszulösen.

Erste Studien, bei denen eine derartige Therapie bei zwei Patientinnen mit Brustkrebs angewandt wurde, und bei denen die Patientinnen auf die Immun-Therapie angesprochen haben, stehen kurz vor dem Abschluss.

## Patentansprüche

1. *Ex vivo-*.Verwendung einer Nukleinsäure, codierend für ein Peptid, das **dadurch gekennzeichnet ist, dass** es vom MUC-1-Gen abgeleitet ist und die Sequenz SEQ ID-Nr.: 2:
Leu Leu Leu Leu Thr Val Leu Thr Val,
aufweist, und die in einem Expressionsvektor enthalten sein kann, zur Transformation oder Transfektion im Rahmen einer Gentherapie.

2. Verwendung einer Nukleinsäure nach Anspruch 1 zur Transformation oder Transfektion von Zellen *in vitro.*

3. Verwendung nach Anspruch 1 oder 2 oder eines Peptides oder einer Nukleinsäure nach Anspruch 1 zur Herstellung eines Arzneimittels zur Auslösung einer Immunreaktion im Zusammenhang mit einer Tumortherapie oder mit einer gegenüber der Entstehung eines Tumors vorbeugenden Behandlung in Kombination mit einem Pan-HLA-DR-bindenden Peptid.

4. Verwendung eines Peptides oder einer Nukleinsäure nach Anspruch 1 zur Herstellung eines Arzneimittels zur Auslösung einer Immunreaktion im Zusammenhang mit einer Tumortherapie oder mit einer gegenüber der Entstehung eines Tumors vorbeugenden Behandlung oder Verwendung nach Anspruch 3, **dadurch gekennzeichnet, daß** mindestens eines der Peptide oder eine der Nukleinsäuren zusammen mit Antigen-präsentierenden Zellen, insbesondere dendritischen Zellen, inkubiert wird.

## Claims

1. *Ex vivo* use of a nucleic acid encoding a peptide being **characterized by** being derived from the MUC-1 gene and comprising the sequence SEQ ID NO.: 2:
Leu Leu Leu Leu Thr Val Leu Thr Val,
that may be present in an expression vector, for transformation or transfection as part of a gene therapy.

2. Use of a nucleic acid according to claim 1 for transformation or transfection of cells *in vitro.*

3. Use according to claim 1 or 2 or of a peptide or of a nucleic acid according to claim 1 for producing a drug to induce an immune reaction in connection with a tumor therapy or with a treatment to prevent the formation of a tumor, in combination with a pan-HLA-DR-binding peptide.

4. Use of a peptide or of a nucleic acid according to claim 1 for producing a drug to induce an immune reaction in connection with a tumor therapy or with a treatment to prevent the formation of a tumor, or use according to claim 3, being **characterized by** at least one of the peptides or one of the nucleic acids being incubated together with antigen-presenting cells, especially dendritic cells.

## Revendications

1. Utilisation ex vivo d'un acide nucléique codant pour un peptide, qui est **caractérisé en ce qu'**il est dérivé du gène MUC-1 et présente la séquence SEQ ID N° :2 :
Leu Leu Leu Leu Thr Val Leu Thr Val
et qui peut être contenu dans un vecteur d'expression pour la transformation ou la transfection dans le cadre d'une thérapie génique.

2. Utilisation d'un acide nucléique selon la revendication 1 pour la transformation ou la transfection de cellules *in vitro.*

3. Utilisation selon la revendication 1 ou 2 ou d'un peptide ou d'un acide nucléique selon la revendication 1 pour la préparation d'un médicament destiné à déclencher une réaction immune en corrélation avec un traitement d'une tumeur ou avec un traitement préventif contre l'apparition d'une tumeur en combinaison avec un peptide liant de Pan-HLA-DR.

4. Utilisation d'un peptide ou d'un acide nucléique selon la revendication 1 pour la préparation d'un médicament destiné à déclencher une réaction immune en corrélation avec un traitement d'une tumeur ou avec un traitement préventif contre l'apparition d'une tumeur ou utilisation selon la revendication 3, **caractérisée en ce qu'**au moins un des peptides ou un des acides nucléiques est incubé avec des cellules présentant des antigènes, notamment des cellules dendritiques.


### SEQUENZPROTOKOLL

<110> Immatics Biotechnologies GmbH
<120> Peptid aus Antigen MUC-1 zur Auslösung einer Immunreaktion gegen Tumorzellen
<130> 4648P108EP
<140>
   <141>
<160> 2
<170> PatentIn Ver. 2.1
<210> 1
   <211> 9
   <212> PRT
   <213> Künstliche Sequenz
<220>
   <223> Beschreibung der künstlichen Sequenz: Fragment aus dem durch das Gen MUC-1 codierten Protein des Menschen
<400> 1
   Ser Thr Ala Pro Pro Val His Asn Val
   1 5
<210> 2
   <211> 9
   <212> PRT
   <213> Künstliche Sequenz
<220>
   <223> Beschreibung der künstlichen Sequenz: Fragment aus dem durch das Gen MUC-1 codierten Protein des Menschen
<400> 2
   Leu Leu Leu Leu Thr Val Leu Thr Val
   1 5